# EUROPEAN PATENT APPLICATION

(11) **EP 1 310 264 A1**
(43) Date of publication of application: **14.05.2003**
(21) Application number: 02024835.7
(22) Date of filing: 07.11.2002
(51) Int. Cl.: A61L 9/03

(54) **Fragrance dispensing device for an oil candle**

(30) Priority: 07.11.2001 US 52944
(71) Applicant: Blyth, Inc., Batavia, Illinois 60510 (US)
(72) Inventor: Wesley, John N., Edison, New Jersey 08817 (US)
(74) Representative: Winkler, Andreas, Dr.

(57) **Abstract**

A liquid fuel candle apparatus with a polymeric element impregnated with a fragrant volatile medium includes a wick with an ignitable end and an absorbent end, a receptacle for holding a quantity of fuel, with the absorbent end of the wick in contact with the fuel, a receptacle having at least a first wall which forms a perimeter, with the ignitable end of the wick extending through the receptacle, and a polymeric element impregnated with a volatile medium disposed within the receptacle perimeter, and in substantially continuous contact with the perimeter wall.

## Description

### BACKGROUND OF THE INVENTION

### Field of the Invention

The invention is directed generally to devices for releasing a fragrant volatile medium. More specifically, the invention is directed to an oil lamp for releasing a fragrance or other volatile medium.

### Background of the Invention

Fragrance candles have become quite popular for both commercial and domestic use. Past devices and methods for combining a fragrance with a candle include solidified, scented wax candles. Such a candle does not burn cleanly and leaves a molten wax pool which can cause burns. Furthermore, once the candle has been depleted it cannot be replenished with a fragance or otherwise be used again. Even if the candle is not in use, the fragrance may be depleted over time due to exposure to the surrounding atmosphere which allows the fragrant medium to evaporate or vaporize,

Other developments include placing a structure that rests on the top of a wax candle around the flame. The structure includes reservoir which holds a volatile liquid that is heated by the flame In addition to the above problems with a wax candle, having a volatile liquid so close to the flame may cause a fire hazard. The candle may easily be tipped with the volatile liquid spilling and possibly catching fire. If the volatile liquid is diluted so as to avoid igniting, it is then less fragrant.

Therefore, liquid-based candles and oil lamps have been developed. These candles use a liquid fuel source, usually an organic fuel such as oil, liquid wax or another oxygenated fuel, that can be replenished when depleted. Such a fuel source is also generally smokeless and clean-burning.

Methods for combining a fragrance with a liquid candle has included using a fragrance oil, or other fragrance liquid, as a fuel for the candle. However, using a fragrant fuel source is neither economical nor effective since the essential oils in the fragrance will be burned up in the combustion process. To partially alleviate this problem, holes or vents are placed in or near the top of the fuel container to allow the fragrance in the oil to escape before combustion. However, leaving the vents open also allows the fuel to evaporate and become prematurely depleted. Furthermore, the strength of the fragrance is minimal and does not circulate very well. This is because the fragrance is generally in the form of a volatile liquid. When a volatile liquid is heated and vaporized, the fragrance is released more intensely. However, the volatile liquid generally does not reach a sufficient temperature to release the fragrance when it is part of the fuel, absent combustion. Furthermore, if the evaporation point of the volatile liquid is at a lower temperature, such as around room temperature, it could prematurely evaporate due to the open vents described above and the fragrance could be prematurely depleted.

In order to alleviate the above problems and still combine a fragrance with a candle, liquid-based candles have been developed which use a secondary fragrance source separate from the fuel. The fragrance source is usually placed proximate the flame of the candle to facilitate heating, thereby vaporizing the volatile liquid embedded in the secondary source. The secondary fragrance source can be heated by radiant heat or conductive heat transfer. The secondary fragrance source can be either the volatile liquid itself. diluted in another liquid, or embedded in some other material such as a porous ceramic or a polymeric material.

Use of a porous ceramic results in the possibility that the ceramic can catch fire. Since the ceramic material is generally porous in order to retain a liquid, it can only adsorb the volatile liquid rather than absorbing the volatile liquid. This allows the fragrance vapor to heat faster, and thus reach its flashpoint faster if too much of the volatile liquid has been added. To avoid catching fire, the ceramic must hold less of the volatile liquid than its capacitance would otherwise allow. This means that the fragrance is not as strong and does not last as long.

While polymeric materials have also been used with liquid candles, there was a cause of concern that such material could melt or catch fire if placed too close to the flame or if having too much contact with a heat conducting element proximate the flame. Thus, past such devices have always minimized the amount of contact that the polymeric material has with a heat conducting element. However, the more heat that is applied to a fragrance element, the more the fragrance is released into the surrounding atmosphere. While polymeric materials have been shown to hold more fragrance than a ceramic material, the intended minimal contact with the heat-conducting element limits the amount of fragrance that can be released. In some cases, the minimal amount of contact requires the size of the material to be smaller than it could be if it were allowed to remain in contact with the heat conducting element.

Therefore, there is a need for a liquid burning candle using a fragrance material external to the fuel source. Furthermore, it is desirable that the fragrance material be a polymeric material that has a higher tolerance for heat. Additionally, the liquid candle and the fragrance material should be combined so as to maximize the transfer of heat from the flame to the fragrance material, thereby releasing the maximum amount of fragrance, but all without reaching the flashpoint of the volatile liquid.

### SUMMARY OF THE INVENTION

Accordingly, the invention is directed to a fragrance assembly for a liquid candle. The fragrance assembly includes a receptacle comprising a perimeter wall; and a fragrant polymeric element disposed within the receptacle, wherein a surface of the polymeric element is in substantially continuous contact with the perimeter wall.

The invention is further directed to a candle which includes a wick having an ignitable end and an absorbent end; a container holding a quantity of fuel, wherein the absorbent end is in contact with the fuel; a receptacle having at least one perimeter wall. wherein the ignitable end extends through a portion of the receptacle; and a polymeric element impregnated with a volatile fragrant medium and disposed within the receptacle, wherein at least a first surface of the polymeric element is in substantially continuous contact with the perimeter wall.

The invention is still further directed to a fragrance candle which includes a wick having an ignitable end and an absorbent end; a container holding a quantity of fuel, wherein the absorbent end is in contact with the fuel; a diathermic receptacle comprising an exterior perimeter wall and an opposing interior wall, wherein the exterior perimeter wall and the interior wall form a channel and the ignitable end extends through a portion of the interior wall; and a polymeric fragrance element is disposed within the channel, with at least one generally vertical edge surface of the fragrance element is in contact with at least one wall of the receptacle. Preferably, the polymeric fragrance element has at least one of an inner edge surface portion in substantially continuous contact with the interior wall and an outer edge surface portion in substantially continuous contact with the exterior wall.

The invention is yet further directed to a method of adding a fragrance material to a liquid candle which has a receptacle that includes a perimeter wall. The method comprises: heating a fragrant thermoplastic material; shaping the heated thermoplastic material to the dimensions to the receptacle to form a fragrant polymeric element in substantially continuous contact with the perimeter wall when placed in the receptacle.

### BRIEF DESCRIPTION OF THE DRAWINGS

These and other features and advantages will become more apparent from a detailed consideration of the invention when taken in conjunction with the drawings in which:
Figure 1 is a cross-sectional side view of one embodiment of a candle with a polymeric fragrance element.
Figure 2 is a magnified view of Figure 1 showing an embodiment of a receptacle for holding the polymeric fragrance element near a wick.
Figure 3 is an exploded view of an embodiment of a candle with a polymeric fragrance element, a cap and a receptacle.
Figure 4 is an exploded view of an embodiment of a candle with a polymeric fragrance element, a pull-tab and a receptacle.

### DETAILED DESCRIPTION OF THE INVENTION

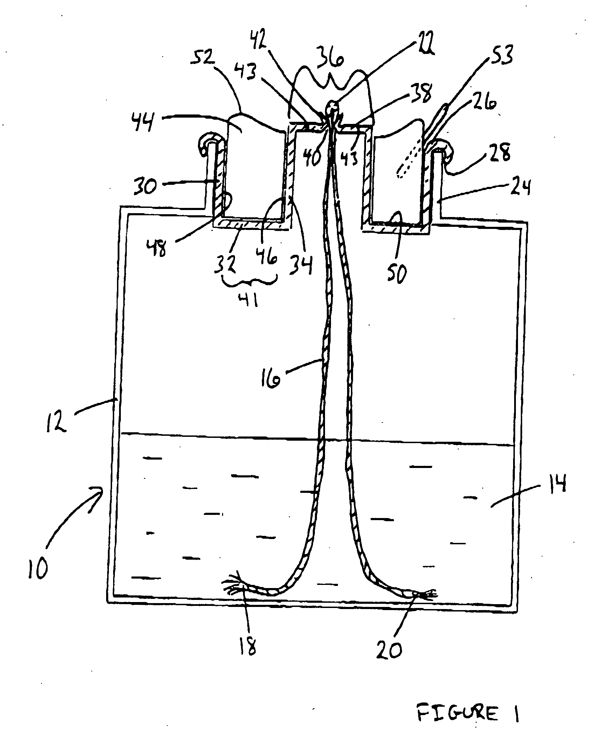

Referring to the drawings, and with specific reference to Figure 1, a liquid-based candle having a fragrance material according to one embodiment of the invention is generally designated 10. The candle 10 includes a container 12 filled with a fuel 14. The container 12 can be of any type of material such as plastic, ceramic, glass, metal or other material suitable to hold fuel without being adversely or unfavorably reactive to the fuel 14. It is preferable that the container 12 be made of a material that will remain relatively cool when the candle 10 is lit so that the candle 10 may be handled without burning or scalding. The container 12 may be either refillable or disposable once the fuel has been depleted. The fuel 14 may be a type of organic fuel, such as a liquid wax, paraffin oil,, mineral oil, alkanes, hydrocarbons or any other suitable combustible fuel source that is able to be absorbed by a wick. Preferably, the fuel 14 is clean-burning and smokeless. Furthermore, the fuel 14 should be odorless and non-toxic so as to not overpower the fragrance.

The candle 10 further includes a wick 16 which can be single or multiple strands with at least one end submerged in the fuel 14 and another end to be ignited. Furthermore, multiple wicks may be used. As shown in Figure 1, the wick 16 is preferably a braided strand with both ends 18, 20 submerged in the fuel 14. Wick ends 18, 20 are frayed to facilitate absorption of the fuel 14. The wick 16 further has a midpoint which forms a loop. This acts as an ignitable end 22 and is exposed at the top of the candle 10. The wick 16 is preferably of a length such that the wick ends 18, 20 rest on the bottom of the container 12 so that virtually all the fuel 14 is utilized. If the candle 10 is a disposable cartridge such that the entire assembly is thrown away once the fuel 14 is depleted, it is desirable to use up as much of the fuel 14 as possible to avoid unnecessary waste. In any case, the wick 16 should be made of a material that is substantially hydrophilic and absorbent with a fuel such as liquid wax.

As indicated in Figure 1, the container 12 includes an upstanding rim 24 at the top of the container 12 which forms an opening, Extending across the opening is a wick holder or receptacle member 26. Preferably the receptacle 26 is formed of a single piece of metal, such as by known metal stamping techniques to shape the piece and metal-spinning procedures to roll and crimp the receptacle 26 onto the rim 24. The metal used to make the receptacle 26 is preferably tin or aluminum. However, any suitable diathermic material will suffice for the receptacle 26, such as glass, inflammable and heat-resistant polymers, ceramics, etc. Furthermore, the container 12 and the receptacle 26 may be formed as an integral unit. Alternatively, the receptacle 26 may be removable from the container 12 for refilling the container 12 with fuel 14 and thus reusing the candle 10.

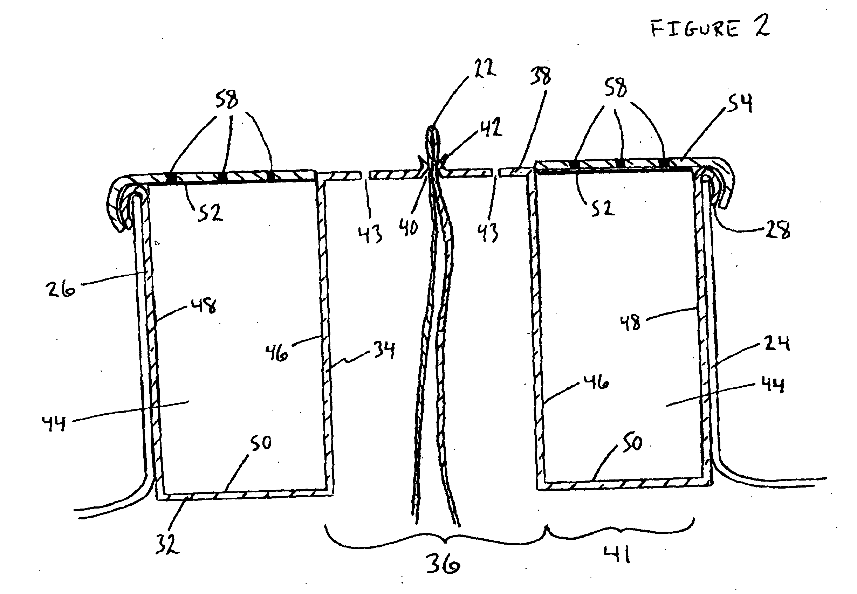

The receptacle 26 is preferably shaped and sized such that it is firmly coupled to the container 12 forming a tight seal. As shown in Figure 2, the outer edge 28 of the receptacle 26 is curled and swaged over the rim 24 to hold the receptacle 26 in place over the opening. The connection may be further sealed by placing a rubber gasket or the like around the rim 24, and having the outer edge 28 curled and swaged over the gasket. The receptacle 26 further has an exterior perimeter wall 30 extending down from the outer edge 28 along the interior of the rim 24 and terminating in a bottom 32. The bottom 32 extends generally radially towards the center of the circle formed by the receptacle 26 and then terminates in an upraised interior wall 34 which forms a column 36 at generally the center of the receptacle 26. Together, walls 30, 34 and bottom 32 form a channel-shape for the receptacle 26. In one embodiment, the receptacle 26 forms an annular channel 41 generally centered about the column 36, However, the channel 41 may be of any size or shape and the walls of the receptacle 30, 34 and the bottom 32 may vary in height, orientation, texture, etc. For example, the column 36 may extend higher than the perimeter wall 30 or either of the walls 30, 34 may vary in height throughout the length of the wall. The walls 30, 34 may also be angled such that they are non-parallel to one another (e.g. the interior wall 34 may form an inverted cone and the perimeter wall 30 may flare out and away from the interior wall 34). The bottom 32 may be ribbed, flat, sloped, etc.

The column 36 terminates in a top 38 having a hole 40 through which the ignitable end 22 of the wick 16 extends. The hole 40 is punched open or otherwise formed so as to cause shearing of the metal. The shearing of the metal forms upraised barbs 42 around the hole 40 which can be crimped onto the ignitable end 22 and hold the wick 16 in place. Other methods of holding the wick 16 in place are also available such as by friction force, a grommet, an adhesive, or other suitable means that are able to withstand a candle flame. Furthermore, the top 38 has one or more vents 43 formed therein defining an air passage between the container 12 and the surrounding atmosphere. These vents 43 provide a safety measure to allow air pressure within the container 12 to escape. As the candle 10 burns, pressure within the container 12 normally builds up. If this pressure is not released, such as by vents 43, it can cause increased capillary action in which too much fuel 14 is forced up the wick 16. In addition, not all of the fuel 14 is combusted and the extra fuel spills out over the candle around the surrounding area. This extra fuel can catch fire and cause a fire hazard. Thus, the vents 43 in the top 38 allow the pressure to escape so that the fuel 14 may travel up the wick 16 at the desired rate.

As mentioned, the exterior perimeter wall 30, the interior wall 34 and the bottom 32 collectively form an integral channel member 41 surrounding the column 36 and the wick 16. Within this channel 41 sits a polymeric fragrance element 44. Any type of polymeric material is suitable, provided that it can withstand the heat from a flame, either by conducted heat and/or radiated heat, without flow (e.g. melting of the material) or flaring (e.g. ignition of volatile components from the material), while controllably releasing a volatile fragrance medium that has been impregnated in the material when heated. That is, the polymeric fragrance element 44 must be formed of a suitable material, such as thermoplastic polymers or polyolcfin matrices, so that it will not melt or thin out while the candle 10 is lit, and so that the polymeric fragrance element 44 will not ignite. Preferably, the polymeric fragrance element 44 is made from a polyamide resin base blended with fragrant oils and formed in a single, solid shape.

The temperatures in and around the polymeric fragrance element may range from approximately 35°C (100°F) at the outer edge 28 of the receptacle 26, up to 100°C (210°F) along the exterior perimeter wall 30 of the receptacle 26, and up to approximately 190°C (374°F) at the ignitable end 22 of the wick 16, The above temperatures are based upon infrared thermograph scans of a candle 10 burning for six hours. The candle 10 used liquid paraffin as a fuel 14, aluminum for the receptacle 26, and a fragrant polypropylene copolymer as the polymeric fragrance element 44. Over a six hour bum time, the polymeric fragance element 44 and surrounding exterior perimeter wall 30 reached a maximum temperature of approximately 99°C (211°F) and plateaued between 92° - 99°C (198° - 211 °F) after only 1.5 hours. Nevertheless, the polymeric fragrance element 44 was successfully able to withstand these temperatures without flow or flaring while controllably releasing the fragrance.

Examples of materials suitable for use as a polymeric fragrance element 44 can be found in U.S, Pat. No. 4,184,099 (Jan. 5, 1980), which is hereby incorporated by reference. That patent discloses a co-solvent free, alcohol free polyamide resin composition that may contain 35-70% by weight of a volatile ingredient, such as perfume oils, and withstand relatively high temperatures of 120°C or more without softening. The polyamide resin is a fatty'polyamide resulting from the reaction of polyamides (e.g diamines and triamines) with relatively high molecular weight dibasic acids (e.g. the condensation products of dimerization of diunsaturated carboxylic acids). For example, the fatty acid may be the condensation product of dimerized linoleic acid and ethylene diamine. These polyamides have molecular weights within the range of about 9,000 up to about 12,000, and maintain their room temperature hardness upon heating until very near the melting or softening temperature within the range of from about 120°C up to about 400°C.

A further description of such a polyamide resin is described in U.S. Pat. No, 3,396,180 (Aug. 6, 1968), which is hereby incorporated by reference. That patent describes a polyamide resin created by reacting ethylene daimine with a polymeric fat acid using one molar equivalent of amine per molar equivalent of carboxyl group at a temperature within the range of 150° - 300°C.

Other examples of polymeric fragrance elements include perfume oils, essential oils, fragrant oils, natural fragrant extracts or other volatile substances as known by those skilled in the art, blended with a polymer selected from the group consisting of low density polyethylenc, high density polyethylene, polypropylene, polypropylene copolymers, ethylene vinyl acetate copolymers, and combinations thereof, or other such suitable materials that do not flow or flare due to heat from the flame.

The volatile fragrance medium impregnated in the polymeric fragrance element 44 is preferably a liquid that at least partly vaporizes upon application of heat; it can be an aesthetic fragrance, an insect repellent, citronella, a medicine, or any other suitable medium. When impregnated in the polymeric frasance element 44, the volatile medium should also preferably undergo minimal vaporization at or below room temperature, i.e. when the candle 10 is not in use, so as to retain most of the remaining fragrance. However, some release of the fragrance is permissible and may be unavoidable; in some uses that may even be desirable such as at restaurants. In any event, the amount of volatile medium in the polymeric fragrance element 44 may vary according to the concentration of fragrance in the volatile medium, the pungency of the fragrance, the desired longevity of the polymeric fragrance element 44, or any other desired aspect. However, it is preferred that the amount of the volatile medium be no more than what the polymeric material can hold without exuding the unvaporized volatile medium onto the surface of the polymeric fragrance element 44, the receptacle 26, or the surrounding area. In other words, the amount of volatile medium in the polymeric fragrance element 44 is preferably no greater than the saturation point of the fragrance in the polymeric material itself.

To form the polymeric fragrance element 44, the polymeric material may be provided in the form of fragrant thermoplastic pellets or other raw material as described above, and heated so as to soften or melt the material for shaping. The softened or melted polymeric material is then formed into the desired shape, and allowed to cool and harden into element 44. The shaping process may be performed in a number of ways. For example, the melted material may be formed in a mold of the desired shape of the element 44. Preferably, the mold would be shaped similar to the channel 41. The formation process may also be done in conjunction with the overall manufacturing process for the candle 10. That is, once the receptacle 26 has been formed, the melted polymeric material can be poured into the channel 41. This permits the outer side and bottom edges of the polymeric fragrance element 44 to be in direct contact, i.e. in substantially continuous contact, with most, if not all, of the surface area of the channel 41. This may be of particular importance if the surfaces of the element 44 are uneven or the shape chosen for the channel 41 has any crevices that would otherwise make it difficult to independently shape and implement the polymeric fragrance element 44 in the channel 41.

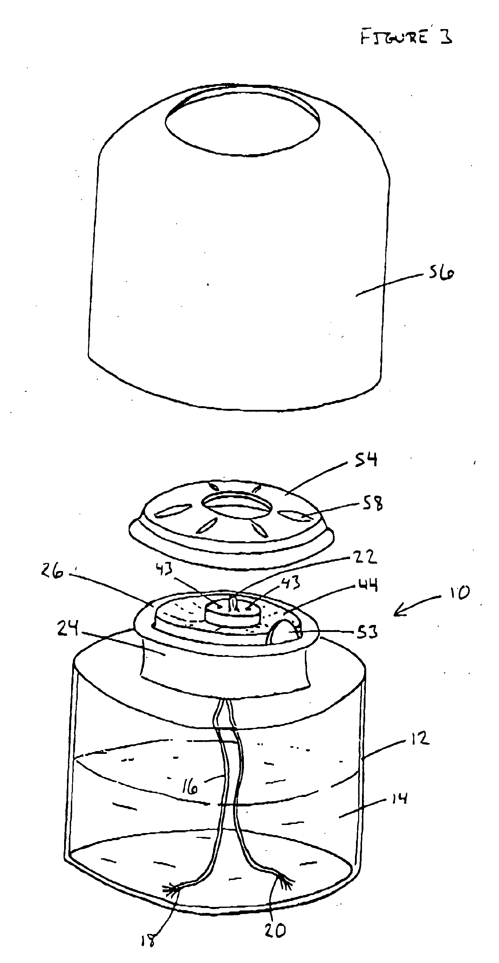

In an alternative embodiment, the softened polymeric material may be rolled into a sheet of a desired thickness and the shape of the element 41 may be stamped out of the sheet. In a further embodiment, the softened material may be extruded and cut to form the desired size and shape. For example, in the case of the annular channel 41 as shown in Figure 3, the softened material may be extruded into the shape of a tube having a cross-section the size and shape of the annular channel 41. The tube may then be cut to the desired thickness to form the polymeric fragrance element 44. Other plastic formation techniques such as spin welding, ultrasonic welding, vibration welding, hot plate welding, injection molding, compression molding, laser cutting or any other suitable techniques may be used to form the polymeric fragrance element 44.

Shaping the polymeric material in the above fashion allows the various edges of the polymeric fragrance element 44 to be in maximum contact with the surfaces of the channel 41 formed in the receptacle 26. Preferably, the polymeric fragrance element 44 is shaped in a ring or amulet around the column 36, and associated wick 16, and is in contact with most of the surface area of the raised walls 30, 34, and the bottom 32. However, any shape for the polymeric fragrance element 44 is suitable provided that it contacts some substantial part of the surfaces of the receptacle 26, so as to receive heat thereby when the ignitable wick end 22 is lit. This may require the walls 30, 34, and hence the channel 41, to be in the same shape as the polymeric fragrance element 44.

The polymeric fragrance element 44 has an inner portion 46, an outer portion 48, a bottom 50 and a top 52. As shown in Figure 1, the inner portion 46 is in direct and continuous contact with the interior wall 34 forming the column 36, and the outer portion 48 is in direct and continuous contact with the exterior wall 34. Furthermore, the bottom 50 of the polymeric fragrance element 44 rests on the bottom 32 of the receptacle 26. Since the receptacle 26 is formed of a diathermic material in this embodiment, heat from a flame is conducted through the material of the receptacle 26 to the polymeric fragrance element 44.

With a majority of the surface area of the polymeric fragrance element 44 in direct contact with the various surface area of the channel 41, the amount of heat conducted within the channel 41 of the receptacle 26 to the polymeric fragrance element 44 is maximized. This in turn heats more of the volatile medium impregnated in the polymeric material to drive out more of the fragrance from element 44, Furthermore, the polymeric fragrance element 44 has more volume than prior devices that had only minimal contact with the heat conducting element. Thus, element 44 can hold more volatile medium to make the fragrance last longer. The polymeric fragrance element 44 may further be friction-fitted into the channel 41. This would further prevent the polymeric fragrance element 44 from falling out if, for example, the candle 10 were tipped or a restaurant patron were trying to pry it out, for example.

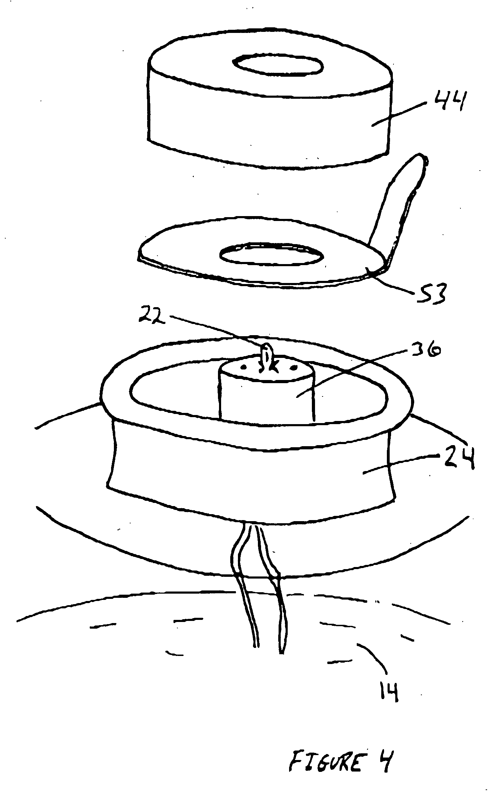

The polymeric fragrance element 44 may further include a pull-tab 53 to aid in removing the element 44 from the channel 41. The pull-tab 53 may be incorporated into the polymeric fragrance element 44 itself as shown in Figure 1, which may be done as part of the manufacturing process, or it may be a separate piece that may extend under most of the polymeric fragrance element 44. Referring to Figure 4, in the case of an annular channel, the pull-tab 53 could be in the shape of a ring that sits within the channel 41 under the clement 44, with a tab extending from the ring and above the rim 24. The pull-tab 53 may be made of thermoplastic, metal or any other suitable material that is able to withstand the heat from the candle flame and the receptacle 26 without melting or otherwise deforming.

As shown in Figure 1, the top 52 of the polymeric fragrance element 44 is not in contact with a heat conductive element. This permits the fragrance to escape up into the surrounding atmosphere. However. as shown in Figures 2 and 3, it is possible to have a heat conductive element extend partially or substantially fully over the top 52, i.e. such as a cap 54 on the receptacle 26, so as to have even more of the surface area of the polymeric fragrance element 44 in contact with the heated receptacle 26. In such a case it may be necessary to have vents 58 formed in the receptacle 26 or in the cap 54 to allow the fragrance to escape. The cap 54 may also be removable so that the polymeric fragrance element 44 can be replaced once the fragrance has been depleted or when a difference fragrance is desired.

The top *52* of the polymeric fragrance element 44 is formed so as to be below the top 38 of the receptacle 26. This is so that the polymeric fragrance element 44 is not in direct contact with the flame on wick end 22. While the polymeric fragrance element 44 has been described as being resistive to conducted or radiated heat, it is not necessarily flame retardant or impervious to melting when in direct contact with a flame. Therefore, the top 52 of the polymeric fragrance element 44 is purposely formed and maintained below the ignitable end 22 of the wick 16 so as to be below the flame. However, it is also possible that only that portion of the top 52 of the polymeric fragrance element 44 nearest the wick end 22 is below the flame. while that portion (of top 52) furthest from the flame rises even to or above the ignitable end 22 of the wick 16, as shown in Figure 1, Thus, in the case of a ring-shaped polymeric fragrance material 44, the top 52 would generally form an inverted cone, or funnel shape, around the wick 16 as seen in Figure 3.

In operation, the wick 16 draws fuel 14 up from the wick ends 18, 20 to the ignitable end 22 via capillary action so as to keep the wick 16 moist with fuel 14 even when not lit. The wick may then be ignited at its ignitable end 22 to produce a flame continuously supplied by the fuel 24 drawn up from the container 12 As the flame burns, heat from the flame is conducted through the receptacle 26. The polymeric fragrance element 44, being in substantial contact with most of the inner surface of the channel 41, is heated via conductive heat from the receptacle 26. Radiative heat from the flame may also aid in heating the polymeric fragrance element 44. The heat activates the volatile liquid within the polymeric fragrance element 44 so as to cause the volatile liquid to vaporize and be released from the element 44, thus releasing the fragrance.

The release of the fragrance is preferably controlled and continuous over a period of time, as opposed to vaporizing all the volatile liquid at once. The amount and rate of release is dependent on the amount of heat applied To the polymeric fragrance element 44. By placing much of the polymeric fragrance element 44 in contact with the surface of the channel 41, as opposed to trying to minimize contact, more heat is applied to the element 44 thereby releasing more of the fragrance. However, the release is still continuous and controlled, Furthermore, since the polymeric fragrance element 44 may be formed to the size of the receptacle 26, the overall size of the element 44 is greater than if contact were minimized. The greater size allows the polymeric fragrance element to hold more volatile liquid and increase its longevity. This helps to offset any possible decrease in the useful life of the polymeric fragrance element 44 due to the increased release of the fragrance.

Further embodiments and variations of the invention are also contemplated. For example, the wick 16 does not have to be at the center of the receptacle surrounded by the fragrance material. Rather, such as for aesthetic reasons, the wick 16 may extend through one side of the receptacle 26 with the channel 41 formed to one side of the wick 16. Thus, only the exterior perimeter wall 30 may be used without the interior wall 34. The container 12 and the receptacle 26 may also be formed as a single, unitary piece.

The polymeric fragrance element 44 could also be disposable, such that when the fragrance is depleted, the polymeric fragrance element 44 can be replaced with another of the same or a different fragrance. Furthermore, the entire assembly can be made to be disposable, such that once the candle 10 is depleted of fuel 14, it is simply replaced with another candle 10. The disposable candle 10 is generally made of inexpensive materials such as plastic for the container 12, and aluminum or tin material for the receptacle 26. As shown in Figure 3, an outer decorative cone or casing 56 that slides over the entire assembly can be provided, thereby covering the candle 10. There will be an opening at the top of the casing 56 through which the flame can extend and fragrance odors can be emitted.

While the invention has been described with reference to specific examples, which are intended to be illustrative only, and not to be limiting of the invention, it will be apparent to those of ordinary skill in the art that changes, additions or deletions may be made to the disclosed embodiments without departing from the spirit and scope of the invention.

## Claims

1. A fragrance assembly for a liquid candle comprising.
a receptacle comprising a perimeter wall; and
a fragrant polymeric element disposed within the receptacle, wherein a first surface of said fragrant polymeric element is in substantially continuous contact with the perimeter wall.

2. The fragrance assembly of claim 1 further comprising a wick having an ignitable end and an absorbent end, wherein the ignitable end extends though a portion of the receptacle.

3. The fragrance assembly of claim 2 further comprising a container comprising an opening, the container holding a quantity of fuel, wherein the absorbent end is in contact with the fuel and the receptacle is coupled to the opening,

4. , The fragrance assembly of claim 3, wherein the receptacle further comprises at least one vent defining an air passage between the container and the surrounding atmosphere.

5. The fragrance assembly of claim 2, wherein the absorbent end comprises first and second absorbent ends, and wherein the wick further comprises a midpoint disposed between the first and second absorbent ends, and the ignitable end comprises a loop formed at the midpoint.

6. The candle of claim 2, wherein at least a portion of the fragrant polymeric element proximate the ignitable end is positioned below the ignitable end.

7. The fragrance assembly of claim 2, wherein the fragrant polymeric element extends circurnferentially around the ignitable end.

8. The fragrance assembly of claim 1, wherein the receptacle further comprises a second wall and the fragrant polymeric element is disposed between the second wall and the perimeter wall.

9. The fragrance assembly of claim 8, wherein a second surface of the fragrant polymeric element is in substantially continuous contact with the second wall.

10. The fragrance assembly of claim 1, wherein the polymeric element is friction-fitted within the receptacle.

11. The fragrance assembly of claim 1, wherein the receptacle comprises a diathermic material.

12. The fragrance assembly of claim 1, wherein the fragrant polymeric element comprises a polypropylene copolymer impregnated with a volatile fragrant medium.

13. , The fragrance assembly of claim 1 further comprising:
a diathermic cap positioned over the fragrant polymeric element; and
one or more vents defining an air passage between the fragrant polymeric element and the surrounding atmosphere.

14. The fragrance assembly of claim 13, wherein a third surface of the fragrant polymeric element is in substantially continuous contact with the diathermic cap.

15. The fragrance assembly of claim 1 further comprising a pull-tab integrated within the fragrant polymeric element to permit removal of the fragrant polymeric element from the receptacle.

16. The fragrance assembly of claim 1 further comprising a pull-tab disposed within the receptacle beneath the fragrant polymeric element.

17. A candle comprising:
a wick having an ignitable end and an absorbent end;
a container holding a quantity of fuel, wherein the absorbent end is in contact with the fuel;
a receptacle comprising at least one perimeter wall, wherein the ignitable end extends through a portion of the receptacle; and
a polymeric element impregnated with a volatile fragrant medium and disposed within the receptacle, wherein at least a first surface of the polymeric element is in substantially continuous contact with the perimeter wall.

18. The candle of claim 17, wherein the receptacle further comprises a second wall and wherein the polymeric element is disposed between the perimeter wall and the second wall.

19. The candle of claim 18, wherein a second surface of the polymeric element is in substantially continuous contact with the second wall.

20. The candle of claim 17, wherein the polymeric clement is friction-fitted within the receptacle.

21. , The candle of claim 17, wherein the receptacle comprises a diathermic material.

22. The candle of claim 17, wherein the polymeric element comprises a polypropylene copolymer impregnated with a volatile fragrant medium.

23. The candle of claim 17, wherein the wick further comprises a second absorbent end in contact with the fuel and a midpoint disposed between the first and second absorbent ends, and the ignitable end comprises a loop formed at the midpoint.

24. The candle of claim 17, wherein at least a portion of the polymeric element proximate the ignitable end is positioned below the ignitable end.

25. The candle of claim 17, wherein the polymeric clement extends circumferentially around the ignitable end.

26. The candle of claim 17, wherein the receptacle further comprises:
a diathermic cap positioned over the polymeric element,
wherein the diathermic cap is in substantially continuous contact with the polymeric element; and
one or more vents defining an air passage between the polymeric element and the surrounding atmosphere.

27. , The candle of claim 17, wherein the receptacle further comprises at least one vent defining an air passage between the container and the surrounding atmosphere.

28. The candle of claim 17 further comprising a pull-tab integrated within the polymeric element.

29. The candle of claim 17 further comprising a pull-tab disposed within the receptacle beneath the polymeric element.

30. A fragrance candle comprising;
a wick having an ignitable end and an absorbent end;
a container holding a quantity of fuel, wherein the absorbent end of the wick is in contact with the fuel;
a diathermic receptacle comprising an exterior perimeter wall and an opposing interior wall, wherein the exterior perimeter wall and the interior wall form a channel, and the ignitable end extends through a portion of the interior wall; and
a polymeric fragrance element disposed within the channel,
wherein at least one generally vertical edge surface of the polymeric fragrance element is in contact with at least one wall of the receptacle.

31. The fragrance candle of claim 30, wherein the polymeric fragrance element has at least one of an inner portion in substantially continuous contact with the interior wall and an outer portion in substantially continuous contact with the exterior perimeter wall.

32. The fragrance candle of claim 30, wherein the polymeric fragrance element comprises a polypropylene copolymer.

33. The fragrance candle of claim 30, wherein the polymeric fragrance element is friction-fitted within the channel.

34. The fragrance assembly of claim 30 further comprising a pull-tab integrated within the polymeric fragrance element.

35. The fragrance assembly of claim 30 further comprising a pull-tab disposed within the receptacle beneath the polymeric fragrance element,

36. A method of adding a fragrance material to a liquid candle that includes a receptacle having a perimeter wall, the method comprising:
heating a fragrant thermoplastic material; and
shaping the heated thermoplastic material to the dimensions of the receptacle so as to form a fragrant polymeric element in substantially continuous contact with the perimeter wall when placed in the receptacle.

37. The method of claim 36 wherein the step of heating the fragrant thermoplastic material is one of softening the thermoplastic material and melting the thermoplastic material.

38. The method of claim 36 wherein the step of shaping the heated thermoplastic material is one of pour molding, injection molding, compression molding, spin welding, ultrasonic welding, vibration welding, hot plate welding, extrusion, stamping and laser cutting.

39. The method of claim 36 further comprising:
extending a wick having an ignitable end through a portion of the receptacle,
wherein a portion of the fragrant polymeric element proximate the position of the ignitable end is below the position of the ignitable end.

40. The method of claim 36 further comprising coupling the receptacle to a fuel container.

41. The method of claim 36 further comprising inserting a pull-tab within the heated thermoplastic material.

42. The method of claim 36 further comprising placing a pull-tab within the receptacle beneath the fragrant polymeric element.
